# FASCICULE DE BREVET EUROPEEN

(11) **EP 1 452 194 B1**
(45) Date de publication et mention de la délivrance du brevet: **19.09.2007**
(21) Numéro de dépôt: 04290222.1
(22) Date de dépôt: 28.01.2004
(51) Int. Cl.: A61M 1/02, A61B 5/15

(54) **Système à poches emballé pourvu de moyens d'identification**
Verpacktes Beutelsystem mit Identifikationsmittel
Packaged bag system with identification means

(30) Priorité: 19.02.2003 FR 0302033
(43) Date de publication de la demande: 01.09.2004
(73) Titulaire: MACO PHARMA, 59420 Mouvaux (FR)
(72) Inventeur: Demay, Sylvie, 59250 Halluin (FR); Goudaliez, Francis, 59155 Faches-Thumesnil (FR); Verpoort, Thierry, 59420 Mouvaux (FR)
(74) Mandataire: Geismar, Thierry

(56) Documents cités:
- EP-A- 1 190 673
- DE-U- 29 518 784
- US-A- 4 886 071
- US-A- 6 113 554
- US-A- 6 123 859
- US-A1- 2002 019 621

## Description

L'invention concerne un ensemble comprenant un emballage et un système à poches pour le prélèvement d'un fluide biologique ainsi qu'un procédé de fabrication d'un tel ensemble.

Pour des raisons d'asepsie, le système à poches est confiné de façon stérile dans l'emballage lors de la fabrication, ledit emballage étant agencé pour pouvoir être ouvert préalablement à l'utilisation dudit système.

L'invention s'applique typiquement au cas où le fluide biologique est du sang total qui doit être prélevé d'un donneur dans une poche de recueil. Pour ce faire, le système à poches comprend, en circuit clos, un dispositif de prélèvement du sang qui est en communication fluidique avec au moins une poche de recueil du sang. En outre, le système comprend un dispositif d'échantillonnage du sang qui est destiné à recevoir une partie du sang prélevé, ledit dispositif comprenant au moins un récipient d'échantillonnage qui est associé de façon dissociable au système.

L'utilisation d'un tel dispositif d'échantillonnage permet d'obtenir, dans chaque récipient, un échantillon de sang, lesdits récipients pouvant ensuite être dissociés du système pour analyser les échantillons, notamment pour réaliser une sérologie, une virologie et une numération.

En particulier, le système à poches peut être utilisé en prélevant les premiers millilitres de sang dans le dispositif d'échantillonnage, ce qui présente un certain nombre d'avantages. Premièrement, cela permet de diminuer le risque de contamination provenant de la présence de bactéries ou d'autres substances étrangères sur la peau du donneur, puisque les premiers millilitres de sang prélevés concernés par cette contamination sont envoyés dans le dispositif d'échantillonnage et non dans la poche de recueil. Deuxièmement, cela permet de réaliser les échantillons avant que la poche ne soit totalement remplie, et par conséquent de ne pas perdre de temps. Enfin, lors du prélèvement, la perte de volume de sang pour le donneur étant compensée par l'apport de plasma, l'hématocrite du sang à analyser serait plus bas si le dispositif d'échantillonnage était rempli après la poche de recueil, et par conséquent la numération serait faussée.

Un problème qui se pose est celui de la traçabilité des échantillons de sang contenu dans les récipients. En effet, les tests réalisés sur les échantillons ayant pour but de connaître les caractéristiques du sang prélevé, il est impératif de pouvoir connaître la provenance des échantillons. A défaut, on pourrait affecter de mauvaises caractéristiques au sang contenu dans une poche de recueil, ce qui, notamment en cas de transfusion du sang à un patient, peut avoir des conséquences graves.

Dans la pratique actuelle, la résolution de ce problème incombe à l'utilisateur des systèmes à poches qui doit identifier, par exemple au moyen d'étiquettes, les récipients d'échantillonnage qu'il utilise pour échantillonner le sang prélevé dans chaque système à poches. A cet effet, l'utilisateur doit coller une étiquette sur la poche de recueil et sur chaque récipient, lesdites étiquettes contenant des informations permettant de connaître la provenance de l'échantillon. En particulier, ces étiquettes contiennent des informations communes qui permettent de relier les récipients d'échantillonnage à la poche de recueil contenant le sang échantillonné.

Une telle pratique est décrite dans le document US 6,113,554 qui concerne un système automatique de prélèvement du sang permettant de recueillir une quantité prédéterminée de sang, dans lequel les différents éléments du système à poche sont identifiés au moyen d'étiquettes portant des codes-barres apposés par l'utilisateur du système à poches.

Cette pratique ne donne pas entièrement satisfaction en ce qu'elle laisse place à une erreur de manipulation de l'utilisateur, erreur qui est d'autant plus probable lorsque l'utilisateur manipule un grand nombre de systèmes à poches.

L'invention a notamment pour but de résoudre ce problème en proposant un système à poches sur lequel est associé de façon dissociable au moins un récipient d'échantillonnage, un moyen d'identification étant disposé, préalablement à l'emballage du système à poches, sur chaque récipient et sur la poche de recueil, les moyens d'identification comprenant des informations permettant d'établir de façon univoque que le ou les récipient(s) et la poche de recueil proviennent du même système à poches.

Ainsi, en intégrant les moyens d'identification lors de la fabrication du système à poches emballé, l'utilisateur se contente d'ouvrir l'emballage pour utiliser le système à poches, et ce sans se préoccuper de la traçabilité des échantillons obtenus.

A cet effet, et selon un premier aspect, l'invention concerne un ensemble comprenant un emballage et un système à poches pour le prélèvement d'un fluide biologique, notamment sanguin, ledit système étant confiné de façon stérile dans ledit emballage qui est agencé pour pouvoir être ouvert préalablement à l'utilisation dudit système, ledit système comprenant un dispositif de prélèvement du fluide qui est en communication fluidique avec au moins une poche de recueil du fluide, et un dispositif d'échantillonnage du fluide à recueillir qui comprend au moins un récipient d'échantillonnage associé de façon dissociable au système, dans lequel la poche de recueil et les récipients d'échantillonnage sont pourvus chacun d'un moyen d'identification qui comprend des informations permettant, après dissociation du récipient d'avec le système à poches, d'établir de façon univoque que le récipient d'échantillonnage et la poche de recueil proviennent du même système à poches.

Selon un deuxième aspect, l'invention concerne un procédé de fabrication d'un tel ensemble, ledit procédé prévoyant, préalablement à l'emballage du système à poches, de pourvoir la poche de recueil et chaque récipient d'un moyen d'identification qui comprend des informations permettant, après dissociation du récipient d'avec le système à poches, d'établir de façon univoque que le récipient d'échantillonnage et la poche de recueil proviennent du même système à poches.

D'autres objets et avantages de l'invention apparaîtront au cours de la description qui suit, faite en référence aux dessins annexés dans lesquels :

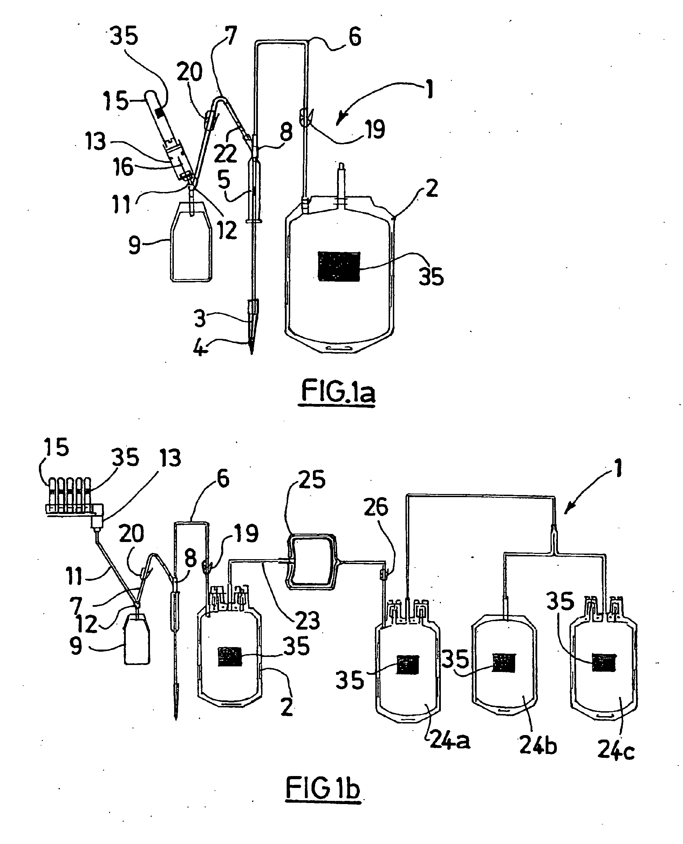

- la figure 1a représente de façon schématique un système à poches pour le prélèvement du sang qui comprend un dispositif d'échantillonnage selon un premier mode de réalisation ;
- la figure 1 b représente de façon schématique un système à poches pour le prélèvement du sang et la séparation des composants sanguins qui comprend un dispositif d'échantillonnage selon un deuxième mode de réalisation ;
- la figure 2 représente de façon schématique le moyen de transfert du dispositif d'échantillonnage représenté sur la figure 1a ;
- les figures 3a et 3b représentent de façon schématique le moyen de transfert de la figure 2 dans lequel un récipient d'échantillonnage est disposé respectivement dans une position à distance et dans une position de transfert ; la figure 3c est une représentation analogue à la figure 3b montrant une variante de réalisation du moyen de transfert ;
- la figure 4 représente de façon schématique un système à poches pour le prélèvement du sang qui comprend un dispositif d'échantillonnage pourvu de plusieurs moyens de transfert selon la figure 2 ;

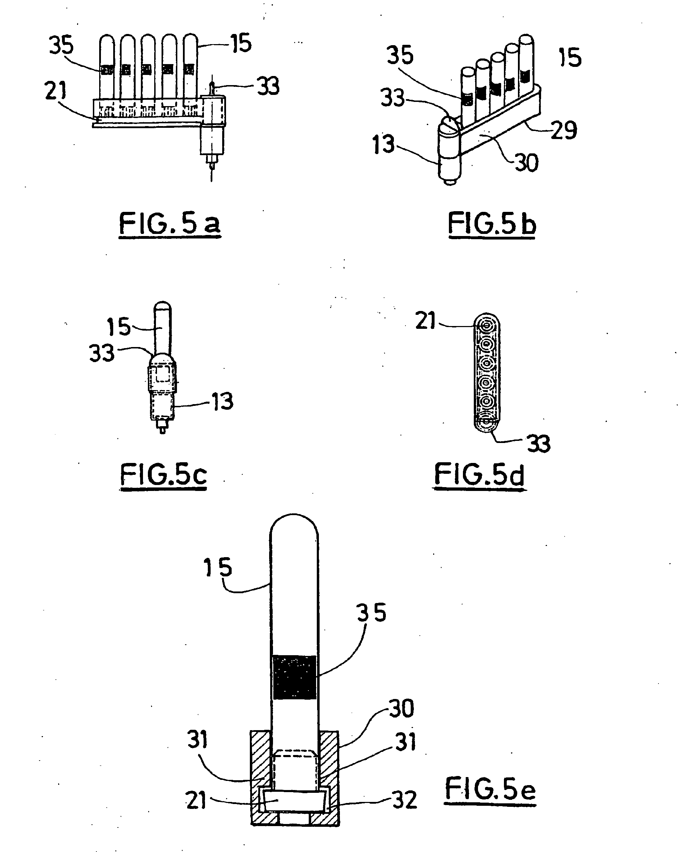

- les figures 5a à 5e représentent de façon schématique le moyen de transfert du dispositif d'échantillonnage de la figure 2, respectivement de face, en perspective, de profil, de dessus et en coupe transversale, les récipients d'échantillonnage étant en position d'attente;
- les figures 6a et 6b représentent de façon schématique le moyen de transfert des figures 5 selon une variante de réalisation, respectivement de face et de profil, les récipients étant en position d'attente ; la figure 6c est une vue analogue à la figure 6b dans laquelle un récipient est en position de transfert ;
- la figure 7 représente un système à poches pour le prélèvement du sang qui comprend un dispositif d'échantillonnage selon un troisième mode de réalisation ;
- les figures 8a et 8b représentent de façon schématique un ensemble comprenant un emballage dans lequel est confiné de façon stérile un système à poches respectivement selon les figures 4 et 1 b.

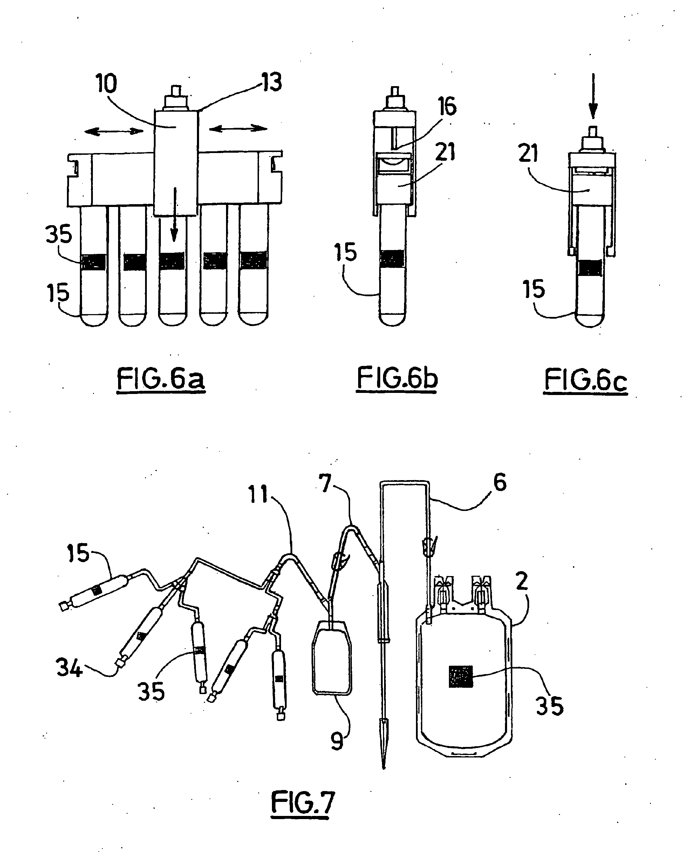

Les figures 1a, 1b et 7 représentent un système à poches 1 comprenant des moyens de prélèvement du fluide auprès d'un donneur et au moins une poche de recueil 2 destinée à recevoir le fluide prélevé, notamment du sang.

Les moyens de prélèvement sont constitués notamment d'une aiguille 3 permettant l'accès à la veine du donneur et d'un capuchon 4 protégeant l'aiguille 3. En outre, un protecteur d'aiguille 5 peut être placé de façon coulissante sur une première tubulure 6 mettant en communication la poche de recueil 2 avec les moyens de prélèvement.

Le système à poches 1 comprend en outre un dispositif d'échantillonnage, qui est en communication fluidique avec la poche de recueil 2 par l'intermédiaire de la première 6 et d'une deuxième tubulure 7 reliées au niveau d'un premier connecteur 8 sous la forme d'une jonction trois voies.

Dans les modes de réalisation représentés, le dispositif d'échantillonnage comprend une poche d'échantillonnage 9 qui est connectée à l'extrémité aval de la deuxième tubulure 7. Les termes aval et amont sont définis par rapport au sens de circulation du sang, du dispositif de prélèvement vers les poches et le dispositif d'échantillonnage.

Le dispositif d'échantillonnage comprend en outre un moyen de transfert 10 du fluide, qui est en communication fluidique avec la poche de recueil 2 par l'intermédiaire de la première 6 et de la deuxième tubulure 7, et éventuellement d'une troisième tubulure 11 reliée à la deuxième tubulure 7 au niveau d'un deuxième connecteur 12 sous la forme d'une jonction trois voies.

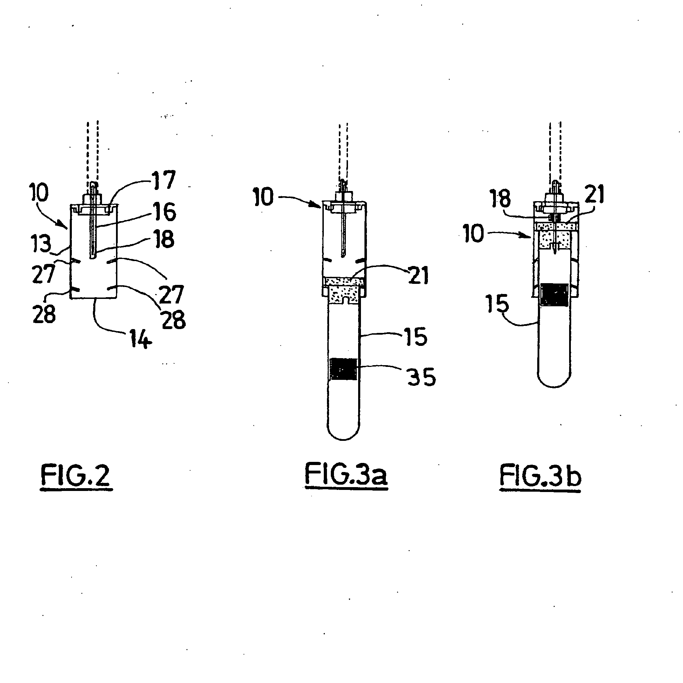

Comme représenté sur la figure 2, le moyen de transfert 10 comprend un guide creux 13, ouvert en partie avant 14 afin de permettre l'introduction d'un récipient d'échantillonnage 15, et une aiguille creuse 16 traversant la partie arrière 17 du guide, de sorte qu'une partie aval de ladite aiguille s'étende à l'intérieur du guide et qu'une partie amont de ladite aiguille 16 s'étende à l'extérieur du guide. Le segment aval de l'aiguille 16 creuse est enfermé dans un fourreau élastique 18. Le segment amont de l'aiguille 16 creuse permet la connexion du moyen de transfert avec le système à poches 1. Un moyen de communication fluidique ou tubulure est alors connecté sur ledit segment amont.

Un premier 19 et un deuxième 20 clamp peuvent être situés respectivement sur la première tubulure 6, en aval du connecteur 8, et sur la deuxième tubulure 7. Les clamps 19, 20 permettent d'orienter le flux du fluide prélevé, soit vers la poche d'échantillonnage 9, le premier clamp 19 est fermé alors que le deuxième clamp 20 est ouvert, soit vers la poche de recueil 2, le deuxième clamp 20 est fermé alors que le premier clamp 19 est ouvert.

Le récipient d'échantillonnage 15 est rempli avec le sang prélevé contenu dans la poche d'échantillonnage 9, lorsque ledit récipient 15 est placé dans la position de transfert, à savoir lorsque l'extrémité aval de l'aiguille 16 est en communication fluidique avec l'intérieur du récipient 15, par perforation d'un élément de fermeture 21 du récipient 15.

Des ouvre-circuits peuvent être prévus au sein du système à poches 1. Notamment un ouvre-circuit 22 peut être situé sur la deuxième tubulure 7 à proximité du premier connecteur 8.

Comme représenté sur la figure 1b, afin de réaliser des étapes de filtration et de séparation ainsi que la déleucocytation des différents constituants du sang, la poche de recueil 2 peut être en communication fluidique, par l'intermédiaire d'une quatrième tubulure 23, avec des poches satellites 24a-c. Un filtre 25 à déleucocyter est situé entre la poche de recueil 2 et une poche satellite 24a. La poche satellite 24a peut être en communication fluidique avec une ou plusieurs autres poches satellites, par exemple la poche satellite 24a peut être en communication fluidique avec deux autres poches satellites 24b,c. Un clamp 26 peut être prévu sur la quatrième tubulure 23 entre la poche de recueil 2 et le filtre 25 à déleucocyter. Selon une réalisation, les poches satellites 24a-c peuvent être pourvues d'un moyen d'identification 35.

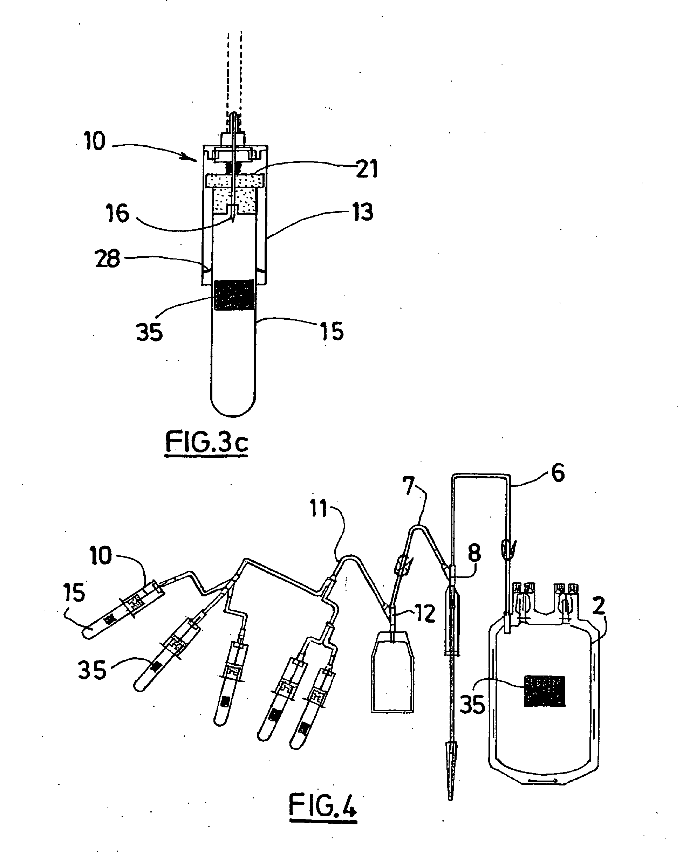

Selon un premier mode de réalisation, le moyen de transfert 10 est pourvu d'un moyen d'association du récipient 15 d'échantillonnage, comme représenté sur la figure 2. Le moyen d'association comprend un premier 27 et un deuxième 28 ensemble de saillies réparties longitudinalement sur la surface interne du guide 13, respectivement à proximité de l'aiguille 16 du guide et à proximité de la partie avant 14 du guide 13. Les saillies sont agencées pour être déformables par coulissement du récipient 15 à l'intérieur du guide 13 de sorte à permettre une association réversible du récipient 15 à l'intérieur du guide 13, et un coulissement du récipient 15 à l'intérieur du guide 13 entre une position d'attente (figure 3a), à distance de l'aiguille 16, et la position de transfert (figure 3b).

Comme représenté sur les figures 2, 3a et 3b, les saillies sont souples, notamment élastiques, et sont déformables réversiblement depuis une position inclinée vers l'avant vers une position inclinée vers l'arrière par contact du récipient 15 lors de son coulissement à l'intérieur du guide 13 dans le sens avant-arrière. Lorsque le récipient 15 est retiré du guide 13, les saillies s'inclinent d'arrière en avant de telle sorte que le récipient n'est pas dissocié de son élément de fermeture 21. Dans la réalisation représentée, le récipient 15 d'échantillonnage comprend un élément de fermeture 21 dont le diamètre est supérieur à celui du corps du récipient 15, c'est lors du passage de l'élément de fermeture 21 que les saillies s'inclinent dans un sens ou dans l'autre.

Selon une variante, représentée sur la figure 3c, les saillies du premier ensemble 27 situé à proximité de l'aiguille 16 sont cassables sous l'effet du coulissement du récipient 15 placé dans la position de transfert. La perforation de l'élément de fermeture 21 est ainsi visible, l'utilisateur peut vérifier que la perforation n'a pas eu lieu préalablement à la prise d'échantillons.

Comme représenté sur la figure 4, plusieurs moyens de transfert 10, dans chacun desquels un récipient 15 d'échantillonnage est associé de façon dissociable, peuvent être connectés au système à poches 1 par l'intermédiaire de la deuxième tubulure 7 ou de la troisième tubulure 11, reliée à la deuxième tubulure 7 par le deuxième connecteur 12. Associer de façon dissociable plusieurs récipients 15 à plusieurs moyens de transfert 10 présente des avantages, d'une part, un gain de temps pour la personne chargée du prélèvement puisqu'elle ne doit pas mettre en place le récipient 15 dans le moyen de transfert 10, et, d'autre part, une diminution du risque d'erreur de traçabilité des dons, puisque cela permet de fixer des étiquettes de traçabilité préalablement à la prise d'échantillons, notamment dès la fabrication.

Selon un deuxième mode de réalisation, le moyen d'association est agencé pour permettre le support de plusieurs récipients 15 à distance du guide 13 dans une position d'attente et leur guidage séquentiel dans le guide 13, comme représenté sur les figures 1 b et 5a à 5e.

Le moyen d'association et le moyen de transfert 10 sont associés par clipsage, par soudage, ou peuvent être moulés en une seule et même pièce.

Le moyen d'association comprend un boîtier 29 associé au guide 13. Ledit boîtier 29 est pourvu d'une jupe 30 dans laquelle l'élément de fermeture 21 des récipients 15 est introduit pour permettre le coulissement longitudinal des récipients 15 dans le boîtier 29 vers le guide 13. La paroi interne de ladite jupe 30 est munie d'une saillie 31 destinée, par interaction avec les éléments de fermeture 21, à empêcher le retrait transversal des récipients 15 du boîtier 29.

La jupe 30 comprend une extrémité ouverte disposée en regard d'une échancrure formée dans le guide, et une extrémité opposée fermée. Dans l'autre axe, une première extrémité ouverte est disposée en regard d'une autre extrémité qui est ouverte de telle sorte que le corps du ou des récipients 15 s'étende au-delà du boîtier 29.

Lors de la fabrication, les récipients 15 d'échantillonnage sont introduits dans le guide 13 par sa partie avant 14 ouverte de sorte que l'élément de fermeture 21 se situe à la hauteur de la rainure 32 du boîtier 29 afin qu'il puisse être glissé dans celle-ci.

Un capuchon 33 est ensuite placé sur le guide 13 permettant de maintenir les récipients 15 dans le boîtier 29 jusqu'à la prise d'échantillons par l'utilisateur du système 1.

Le boîtier peut être de taille variable de sorte à contenir de deux à dix récipients 15. Le nombre de récipients 15 utilisés varie selon les législations, en France, notamment, cinq récipients 15 sont utilisés pour la réalisation des analyses courantes.

Lors de la prise d'échantillons, la personne chargée du prélèvement retire le capuchon 33 du guide 13, fait glisser les récipients 15 jusqu'au guide 13, puis les introduit de sorte que, par perforation d'un élément de fermeture 21 du récipient 15, l'extrémité aval de l'aiguille 16 est en communication fluidique avec l'intérieur du récipient 15. Après qu'un récipient 15 a été rempli, l'utilisateur le retire du guide 13. Dans un exemple de réalisation, le capuchon 33 peut être muni d'un élément d'inviolabilité, tel qu'une languette qui est rompue lors de la première ouverture, de sorte à pouvoir identifier la première manipulation du bouchon 33.

Selon une variante, représentée sur les figures 6a à 6c, le moyen de transfert 10 peut coulisser sur le moyen d'association, de sorte qu'il peut être placé à la hauteur de chaque récipient 15. Lorsque le moyen de transfert 10 est placé à la hauteur d'un récipient 15, l'utilisateur peut alors déplacer ledit moyen de transfert 10 transversalement de sorte que l'élément de fermeture 21 soit perforé par l'aiguille 16. Pour que le moyen de transfert 10 puisse coulisser sur le moyen d'association, deux échancrures opposées sont alors formées dans le guide 13.

Comme représenté sur la figure 1b, le moyen de transfert 10 associant plusieurs récipients 15 d'échantillonnage peut être connecté à un système à poches 1 par l'intermédiaire de la deuxième tubulure 7 et éventuellement de la troisième tubulure 11.

Selon un troisième mode de réalisation, représenté sur la figure 7, le ou les récipients d'échantillonnage peuvent être souples, préformés et connectés à une tubulure du système à poches, notamment à la deuxième tubulure 7.

Le ou les récipients 15 d'échantillonnage sont remplis, simultanément ou successivement, avec le sang prélevé contenu dans la poche 9 d'échantillonnage.

Le remplissage se fait par pression mécanique sur le récipient 15 d'échantillonnage, l'air contenu dans le récipient 15 préformé est chassé dans la poche 9 d'échantillonnage, tandis que le sang contenu dans la poche 9 est chassé dans le récipient 15.

Après la collecte d'échantillons, le récipient 15 d'échantillonnage est hermétiquement soudé et détaché du système à poches.

Un septum 34 peut être prévu sur l'extrémité du récipient 15 opposée à celle où l'on réalise ladite soudure. De cette façon, après soudure, on obtient un ou plusieurs récipients 15 d'échantillonnage compatibles avec les automates d'analyses. Afin de réaliser un certain nombre d'analyses, le récipient 15 peut alors être placé dans un réceptacle de l'automate, une aiguille de l'automate vient percer le septum 34.

Avec les systèmes à poches pour le prélèvement du sang connus, la personne chargée du prélèvement doit identifier par un marquage, la poche de recueil 2 et le ou les récipients 15 d'échantillons correspondant à un même don.

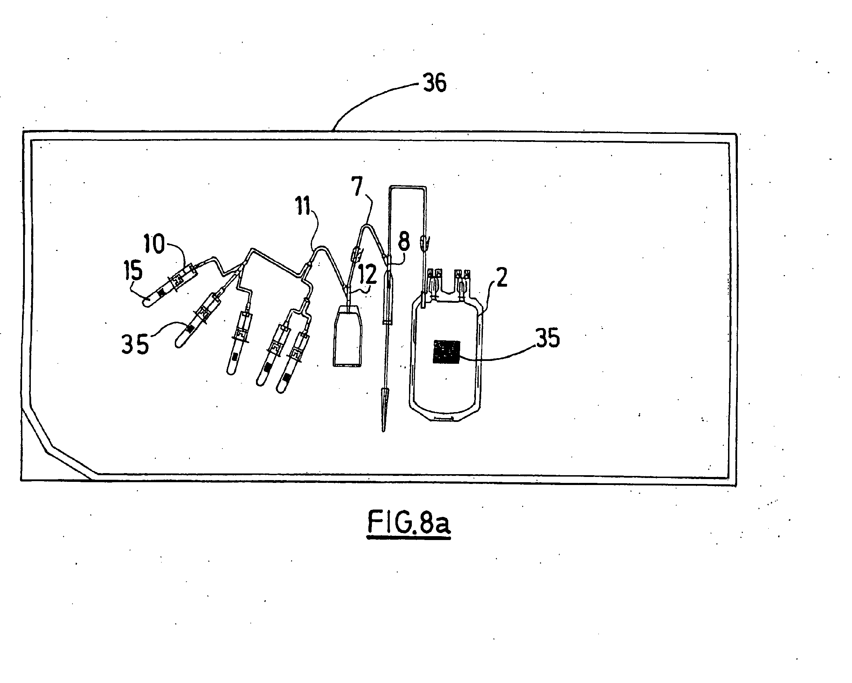

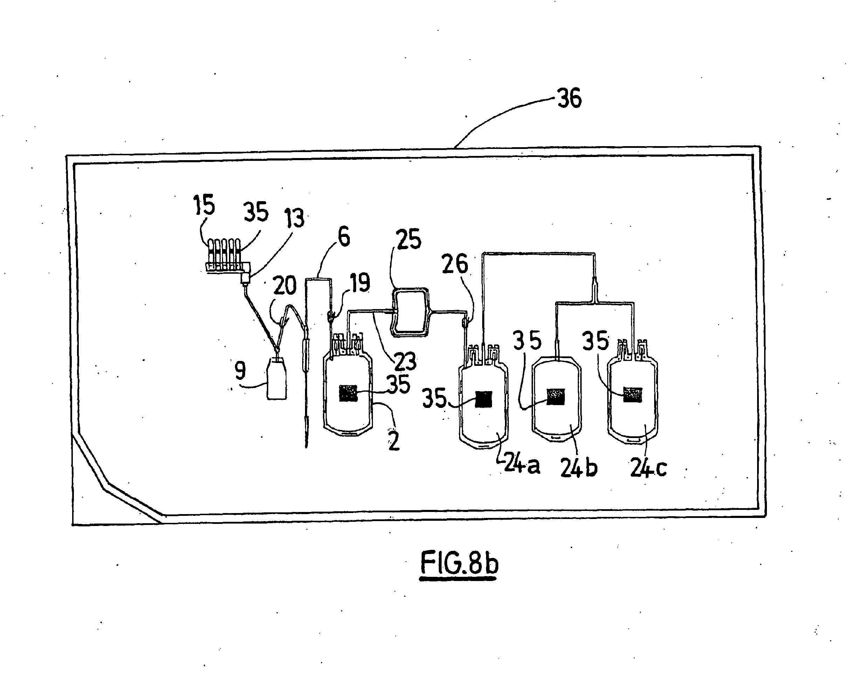

Selon l'invention, la possibilité d'erreur de traçabilité de ces dons est considérablement réduite puisque, comme représenté sur les figures 8a et 8b, le ou les récipients 15 d'échantillonnage et la poche de recueil 2 sont associés de façon dissociable dès la fabrication. En outre, dès la fabrication, la poche de recueil 2 et le ou les récipients 15 d'échantillonnage, ainsi que les éventuelles poches satellites 24a-c, sont pourvus chacun d'un moyen d'identification 35, par exemple au moyen d'une étiquette autocollante à codes barres, qui comprend des informations permettant, après dissociation du récipient d'avec le système à poches 1, d'établir de façon univoque que le récipient 15 d'échantillonnage et la poche de recueil 2, ainsi que les éventuelles poches satellites 24a-c, proviennent du même système à poches 1. Le système à poches 1 selon l'invention peut être emballé dans un emballage 36 souple et transparent. Le système à poches 1 et l'emballage 36 forment ainsi un ensemble.

Le procédé de fabrication d'un tel ensemble prévoit, préalablement à l'emballage du système à poches 1, de pourvoir la poche de recueil 2 et chaque récipient 15 d'un moyen d'identification qui comprend des informations permettant, après dissociation du récipient d'avec le système à poches 1, d'établir de façon univoque que le récipient 15 d'échantillonnage et la poche de recueil 2 proviennent du même système à poches 1. Dans l'exemple de réalisation prévoyant une étiquette 35 pourvue d'un code barres, ledit code barres peut être imprimé sur l'étiquette 35 postérieurement à son collage.

Selon un premier mode de réalisation, le procédé prévoit de disposer le système à poches 1 stérilisé dans l'emballage 36, puis éventuellement de pasteuriser l'ensemble.

Selon un deuxième mode de réalisation, le procédé prévoit de disposer le système à poches 1 dans l'emballage 36, puis de stériliser l'ensemble.

Selon un troisième mode de réalisation, le procédé prévoit de stériliser le système à poches dépourvu de récipients 15, d'associer les récipients 15 au système à poches, d'emballer ledit système puis éventuellement de pasteuriser l'ensemble. Ce mode de réalisation est particulièrement adapté au cas où les récipients d'échantillonnage 15 contiennent des réactifs sensibles à l'étape de stérilisation. Par ailleurs, les éléments de fermeture 21 des récipients d'échantillonnage 15 peuvent également être sensibles à l'étape de stérilisation.

## Revendications

1. Ensemble comprenant un emballage (36) et un système à poches (1) pour le prélèvement d'un fluide biologique, notamment sanguin, ledit système étant confiné de façon stérile dans ledit emballage qui est agencé pour pouvoir être ouvert préalablement à l'utilisation dudit système, ledit système comprenant un dispositif de prélèvement du fluide qui est en communication fluidique avec au moins une poche de recueil (2) du fluide, et un dispositif d'échantillonnage du fluide à recueillir qui comprend au moins un récipient d'échantillonnage (15) associé de façon dissociable au système (1), ledit ensemble étant **caractérisé en ce que** la poche de recueil (2) et les récipients d'échantillonnage (15) sont pourvus chacun d'un moyen d'identification (35) qui comprend des informations permettant, après dissociation du récipient (15) d'avec le système à poches (1), d'établir de façon univoque que le récipient d'échantillonnage (15) et la poche de recueil (2) proviennent du même système à poches (1).

2. Ensemble selon la revendication 1, **caractérisé en ce que** la poche de recueil (2) est en communication fluidique avec le dispositif de prélèvement par l'intermédiaire d'une première tubulure (6) sur laquelle est connecté le dispositif d'échantillonnage par l'intermédiaire d'une deuxième tubulure (7).

3. Ensemble selon la revendication 2, **caractérisé en ce que** le dispositif d'échantillonnage comprend une poche d'échantillonnage (9) qui est connectée à l'extrémité aval de la deuxième tubulure (7).

4. Ensemble selon l'une quelconque des revendications 1 à 3, **caractérisé en ce que** le dispositif d'échantillonnage comprend au moins un moyen de transfert (10) du fluide depuis le système à poches (1) dans un ou des récipients d'échantillonnage (15).

5. Ensemble selon la revendication 4, **caractérisé en ce que** le moyen de transfert (10) comprend un guide creux (13) qui est ouvert en partie avant (14) pour permettre l'introduction d'un récipient d'échantillonnage (15), et une aiguille creuse (16) traversant la partie arrière (17) du guide de sorte qu'une partie aval de ladite aiguille s'étende à l'intérieur du guide et qu'une partie amont de ladite aiguille s'étende à l'extérieur du guide (13), ladite aiguille étant en communication fluidique avec le système à poches (1), ledit guide étant agencé pour, par translation, permettre la disposition du récipient (15) dans une position de transfert dans laquelle, par perforation d'un élément de fermeture (21) du récipient (15), l'extrémité aval de l'aiguille (16) est en communication fluidique avec l'intérieur du récipient (15).

6. Ensemble selon la revendication 5, **caractérisé en ce que** le guide (13) comprend des moyens d'association du récipient (15) introduit, lesdits moyens étant agencés pour permettre une association réversible du récipient (15) à l'intérieur du guide (13) et un coulissement du récipient (15) à l'intérieur du guide (13) entre une position à distance de l'aiguille (16) et la position de transfert.

7. Ensemble selon la revendication 6, **caractérisé en ce que** les moyens d'association comprennent un premier (27) et un deuxième (28) ensemble de saillies déformables par coulissement du récipient (15), lesdits ensembles étant répartis longitudinalement sur la face interne du guide (13).

8. Ensemble selon la revendication 7, **caractérisé en ce qu'**au moins certaines saillies sont souples, notamment élastiques, et sont déformables réversiblement depuis une position inclinée vers l'avant vers une position inclinée vers l'arrière par contact du récipient (15) lors de son coulissement à l'intérieur du guide (13) dans le sens arrière-avant, respectivement avant-arrière.

9. Ensemble selon la revendication 7 ou 8, **caractérisé en ce que** les saillies du premier ensemble (27) qui est situé à proximité de l'aiguille (16) sont cassables sous l'effet de la déformation.

10. Ensemble selon l'une quelconque des revendications 4 à 9, **caractérisé en ce qu'**il comprend plusieurs moyens de transfert (10) dans chacun desquels un récipient (15) est associé de façon dissociable, lesdits moyens étant connectés au système par l'intermédiaire de la deuxième tubulure (7) ou d'une troisième tubulure (11).

11. Ensemble selon la revendication 5, **caractérisé en ce que** le moyen de transfert (10) est pourvu d'un moyen d'association de plusieurs récipients (15) au voisinage du guide (13), ledit moyen étant agencé pour permettre le support des récipients (15) à distance du guide (13) et leur guidage séquentiel dans le guide (13) pour permettre leur disposition dans la position de transfert.

12. Système selon la revendication 11, **caractérisé en ce que** le guide (13) est muni d'un capuchon (33) qui est pourvu d'un élément d'inviolabilité.

13. Ensemble selon la revendication 11, **caractérisé en ce qu'**il comprend un moyen de transfert (10) associant plusieurs récipients (15), ledit moyen étant connecté au système par l'intermédiaire de la deuxième tubulure (7) ou d'une troisième tubulure (11).

14. Ensemble selon l'une quelconque des revendications 1 à 3, **caractérisé en ce que** la tubulure (7, 11) connectant le dispositif d'échantillonnage au système à poches (1) est soudable et sécable, ledit dispositif comprenant au moins un récipient (15) dont l'orifice d'entrée est connecté à l'extrémité aval de ladite tubulure (7, 11).

15. Ensemble selon la revendication 14, **caractérisé en ce que** les récipients d'échantillonnage (15) sont souples.

16. Ensemble selon l'une quelconque des revendications 1 à 15, **caractérisé en ce que** le moyen d'identification est formé d'une étiquette (35) pourvue d'un code barres, cette étiquette (35) étant collée sur la poche de recueil (2) et sur chacun des récipients d'échantillonnage (15).

17. Ensemble selon l'une quelconque des revendications 1 à 16, **caractérisé en ce que** l'emballage (36) est souple et transparent.

18. Ensemble selon l'une quelconque des revendications 1 à 17, **caractérisé en ce que** la poche de recueil (2) est en communication fluidique, par l'intermédiaire d'une quatrième tubulure (23), avec des poches satellites (24a-c), lesdites poches satellites.étant pourvues d'un moyen d'identification (35).

19. Procédé de fabrication d'un ensemble selon l'une quelconque des revendications 1 à 18, ledit procédé prévoyant, préalablement à l'emballage du système à poches (1), de pourvoir la poche de recueil (2) et chaque récipient (15) d'un moyen d'identification (35) qui comprend des informations permettant, après dissociation du récipient (15) d'avec le système à poches (1), d'établir de façon univoque que le récipient (15) d'échantillonnage et la poche de recueil (2) proviennent du même système à poches (1).

20. Procédé selon la revendication 19, **caractérisé en ce qu'**il prévoit de disposer le système à poches (1) stérilisé dans l'emballage (36), puis éventuellement de pasteuriser l'ensemble.

21. Procédé selon la revendication 19, **caractérisé en ce qu'**il prévoit de disposer le système à poches (1) dans l'emballage (36), puis de stériliser l'ensemble.

22. Procédé selon la revendication 19, **caractérisé en ce qu'**il prévoit de stériliser le système à poches (1) dépourvu de récipients (15), d'associer les récipients (15) au système à poches, d'emballer ledit système puis éventuellement de pasteuriser l'ensemble.

## Claims

1. Assembly comprising a packaging (36) and a system of bags (1) for collecting a biological fluid, in particular blood, said system being confined in a sterile manner in said packaging which is arranged to be capable of being opened prior to using said system, said system comprising a fluid collection device in fluidic communication with at least one fluid collection bag (2), and a device for sampling the fluid to be collected, which comprises at least one sampling container (15) removably associated with the system (1), said assembly being **characterised in that** the collection bag (2) and the sampling containers (15) are each provided with identification means (35) comprising information that makes it possible, in an unequivocal manner and after the container (15) has been removed from the system of bags (1), to establish that the sampling container (15) and the collection bag (2) come from the same bag system (1).

2. Assembly according to claim 1, **characterised in that** the collection bag (2) is in fluidic communication with the collection device by means of a first tube (6) to which the sampling device is connected by means of a second tube (7).

3. Assembly according to claim 2, **characterised in that** the sampling device comprises a sampling bag (9) which is connected to the downstream end of the second tube (7).

4. Assembly according to any one of the claims from 1 to 3, **characterised in that** the sampling device comprises at least one means (10) of transferring the fluid from the system of bags (1) to one or more sampling containers (15).

5. Assembly according to claim 4, **characterised in that** the transfer means (10) comprise a hollow guide (13) which is open at the front end (14) to allow the insertion of a sampling container (15), and a hollow needle (16) passing through the rear part (17) of the guide so that a downstream part of said needle extends inside the guide and an upstream part of said needle extends outside the guide (13), said needle being in fluidic communication with the system of bags (1), said guide being arranged so as to allow the container (15) to be placed, by translation, in a transfer position in which, by perforating a closing element (21) of the container (15), the downstream end of the needle (16) is in fluidic communication with the inside of the container (15).

6. Assembly according to claim 5, **characterised in that** the guide (13) comprises means, for association of the inserted container (15), said means being arranged to allow reversible association of the container (15) inside the guide (13) and sliding of the container (15) inside the guide (13) between a position in which it is separated from the needle (16) and the transfer position.

7. Assembly according to claim 6, **characterised in that** the association means comprise a first (27) and second (28) series of protrusions which can deform when the container (15) slides, said protrusions being distributed longitudinally across the inner surface of the guide (13).

8. Assembly according to claim 7, **characterised in that** at least some of the protrusions are flexible, elastic in particular, and are reversibly deformable between a forward-tilted position and a backward-tilted position by contact with the container (15) during its sliding inside the guide (13) in the rear-to-front and front-to-rear directions respectively.

9. Assembly according to claim 7 or 8, **characterised in that** the protrusions of the first series (27) which is located near the needle (16) can break under the effect of deformation.

10. Assembly according to any one of the claims from 4 to 9, **characterised in that** it comprises several transfer means (10) in each of which a container (15) is removably associated, said means being connected to the system by means of the second tube (7) or of a third tube (11).

11. Assembly according to claim 5, **characterised in that** the transfer means (10) are equipped with means for associating several containers (15) in the proximity of the guide (13), said means being arranged to support containers (15) at a distance from the guide (13) and sequentially to guide them in the guide (13) in order to allow them to be placed in the transfer position.

12. System according to claim 11, **characterised in that** the guide (13) is equipped with a cap (33) which is equipped with a tamper-proof element.

13. Assembly according to claim 11, **characterised in that** it comprises transfer means (10) associating several containers (15), said means being connected to the system by means of the second tube (7) or of a third tube (11).

14. Assembly according to any one of the claims from 1 to 3, **characterised in that** the tube (7, 11) connecting the sampling device to the system of bags (1) is weldable and breakable, said device comprising at least one container (15), the inlet orifice of which is connected to the downstream end of said tube (7, 11).

15. Assembly according to claim 14, **characterised in that** the sampling containers (15) are flexible.

16. Assembly according to any one of the claims from 1 to 15, **characterised in that** the identification means consist of a label (35) equipped with a bar code, said label (35) being affixed to the collection bag (2) and to each sampling container (15).

17. Assembly according to any one of the claims from 1 to 16, **characterised in that** the packaging (36) is flexible and transparent.

18. Assembly according to any one of the claims from 1 to 17, **characterised in that** the collection bag (2) is in fluidic communication, by means of a fourth tube (23), with satellite bags (24a-c), said satellite bags being equipped with identification means (35).

19. Method of manufacturing an assembly according to any one of the claims from 1 to 18, said method providing, prior to packaging the bag system (1), for equipping the collection bag (2) and each container (15) with identification means (35) comprising information that makes it possible, in an unequivocal manner and after the container (15) has been removed from the system of bags (1), to establish that the sampling container (15) and the collection bag (2) come from the same bag system (1).

20. Method according to claim 19, **characterised in that** it provides for placing the sterilised system of bags (1) in the packaging (36), then potentially pasteurising the assembly.

21. Method according to claim 19, **characterised in that** it provides for placing the system of bags (1) in the packaging (36), then sterilising the assembly.

22. Method according to claim 19, **characterised in that** it provides for sterilising the system of bags (1) without containers (15), associating the containers (15) to the system of bags, packaging said system then potentially pasteurising the assembly.

## Patentansprüche

1. Einheit bestehend aus einer Verpackung (36) und einem Beutelsystem (1) für die Entnahme einer biologischen Flüssigkeit, insbesondere Blut, wobei das genannte System steril in der genannten Verpackung eingeschlossen ist, die so gestaltet ist, dass sie vor der Benutzung des genannten Systems geöffnet werden kann, wobei das genannte System eine Entnahmevorrichtung der Flüssigkeit umfasst, die strömungstechnisch in Verbindung steht mit mindestens einem Auffangbeutel (2) der Flüssigkeit, sowie eine Probenahme-Vorrichtung für die aufzufangende Flüssigkeit, die mindestens einen trennbar mit dem System (1) verbundenen Probenahme-Behälter (15) aufweist, wobei die genannte Einheit **dadurch gekennzeichnet ist, dass** der Auffangbeutel (2) und die Probenahme-Behälter (15) jeweils ein Identifikationsmittel (35) umfassen, das Informationen enthält, die es nach der Trennung des Behälters (15) von dem Beutelsystem (1) ermöglichen, eindeutig festzustellen, dass der Probenahme-Behälter (15) und der Auffangbeutel (2) zu dem gleichen Beutelsystem (1) gehören.

2. Einheit gemäß Anspruch 1, **dadurch gekennzeichnet, dass** der Auffangbeutel (2) strömungstechnisch mit der Entnahmevorrichtung über einen ersten Stutzen (6) in Verbindung steht, an dem die Probenahme-Vorrichtung über einen zweiten Stutzen (7) angeschlossen ist.

3. Einheit gemäß Anspruch 2, **dadurch gekennzeichnet, dass** die Probenahme-Vorrichtung einen Probenahme-Beutel (9) umfasst, der am nachgelagerten Ende des zweiten Stutzens (7) angeordnet ist.

4. Einheit gemäß einem der Ansprüche 1 bis 3, **dadurch gekennzeichnet, dass** die Probenahme-Vorrichtung mindestens ein Transfermittel (10) der Flüssigkeit von dem Beutelsystem (1) in einen oder mehrere Probenahme-Behälter (15) umfasst.

5. Einheit gemäß Anspruch 4, **dadurch gekennzeichnet, dass** das Transfermittel (10) einen hohlen Führer (13) umfasst, der am vorderen Teil (14) offen ist, um die Einführung eines Probenahme-Behälters (15) zu ermöglichen, sowie eine Hohlnadel (16), die das hintere Teil (17) des Führers durchquert, so dass sich ein nachgelagerter Teil der genannten Nadel innerhalb des Führers und ein vorgelagerter Teil der genannten Nadel außerhalb des Führers (13) erstreckt, wobei die genannte Nadel strömungstechnisch mit dem Beutelsystem (1) in Verbindung steht, wobei der Führer gestaltet ist, um durch Translation die Anordnung des Behälters (15) in einer Transferposition zu ermöglichen, in der das nachgelagerte Ende der Nadel (16) durch Perforierung eines Verschlusselements (21) des Behälters (15) strömungstechnisch mit dem Inneren des Behälters (15) in Verbindung steht.

6. Einheit gemäß Anspruch 5, **dadurch gekennzeichnet, dass** der Führer (13) Verbindungsmittel des eingeführten Behälters (15) umfasst, wobei die genannten Mittel gestaltet sind, um eine reversible Verbindung des Behälters (15) innerhalb des Führers (13) und ein Verschieben des Behälters (15) innerhalb des Führers (13) zwischen einer von der Nadel (16) entfernten Position und der Transferposition zu ermöglichen.

7. Einheit gemäß Anspruch 6, **dadurch gekennzeichnet, dass** die Verbindungsmittel einen ersten (27) und einen zweiten (28) Satz von durch Verschieben des Behälters (15) deformierbaren Vorsprüngen umfassen, wobei die genannten Sätze in Längsrichtung über die Innenseite des Führers (13) verteilt sind.

8. Einheit gemäß Anspruch 7, **dadurch gekennzeichnet, dass** mindestens einige Vorsprünge flexibel, insbesondere elastisch und demnach reversibel deformierbar sind ab einer nach―vorne geneigten Position in eine nach hinten geneigte Position, durch Kontakt des Behälters (15) bei seinem Verschieben innerhalb des Führers (13) in Richtung von hinten nach vorne bzw. von vorne nach hinten.

9. Einheit gemäß Anspruch 7 oder 8, **dadurch gekennzeichnet, dass** die Vorsprünge des in der Nähe der Nadel (16) angeordneten ersten Satzes (27) durch die Deformationswirkung zerbrechlich sind.

10. Einheit gemäß einem der Ansprüche 4 bis 9, **dadurch gekennzeichnet, dass** sie mehrere Transfermittel (10) umfasst, in denen jeweils ein Behälter (15) trennbar verbunden ist, wobei die genannten Mittel über den zweiten Stutzen (7) oder einen dritten Stutzen (11) an dem System angeschlossen sind.

11. Einheit gemäß Anspruch 5, **dadurch gekennzeichnet, dass** das Transfermittel (10) ein Verbindungsmittel (15) mehrerer Behälter (15) in der Nähe des Führers (13) aufweist, wobei das genannte Mittel gestaltet ist, um Behälter (15) in Entfernung des Führers (13) zu tragen und ihre fortlaufende Führung in dem Führer (13) zu ermöglichen, um sie in die Transferposition zu versetzen.

12. System gemäß Anspruch 11, **dadurch gekennzeichnet, dass** der Führer (13) eine Kappe (33) aufweist, die ein Unversehrtheitselement umfasst.

13. Einheit gemäß Anspruch 11, **dadurch gekennzeichnet, dass** sie ein mehrere Behälter (15) verbindendes Transfermittel (10) umfasst, wobei das genannte Mittel über den zweiten Stutzen (7) oder einen dritten Stutzen (11) an dem System angeschlossen ist.

14. Einheit gemäß einem der Ansprüche 1 bis 3, **dadurch gekennzeichnet, dass** der die Probenahme-Vorrichtung an dem Beutelsystel (1) anschließende Stutzen (7, 11) schweißbar und teilbar ist, wobei die Vorrichtung mindestens einen Behälter (15) umfasst, dessen Eingangsöffnung an dem vorgelagerten Ende des besagten Stutzens (7, 11) angeschlossen ist.

15. Einheit gemäß Anspruch 14, **dadurch gekennzeichnet, dass** die Probenahme-Behälter (15) flexibel sind.

16. Einheit gemäß einem der Ansprüche 1 bis 15, **dadurch gekennzeichnet, dass** das Identifikationsmittel aus einem mit einem Barcode versehenen Etikett (35) besteht, wobei dieses Etikett auf den Auffangbeutel (2) und auf jeden Probenahme-Behälter (15) geklebt ist.

17. Einheit gemäß einem der Ansprüche 1 bis 16, **dadurch gekennzeichnet, dass** die Verpackung (36) flexibel und transparent ist.

18. Einheit gemäß einem der Ansprüche 1 bis 17, **dadurch gekennzeichnet, dass** der Auffangbeutel (2) strömungstechnisch über einen vierten Stutzen (23) mit Satellitenbeuteln (24a-c) in Verbindung steht, wobei die genannten Satellitenbeutel ein Identifikationsmittel (35) aufweisen.

19. Herstellungsverfahren einer Einheit gemäß einem der Ansprüche 1 bis 18, wobei das genannte Verfahren vor der Verpackung des Beutelsystems (1) vorsieht, den Auffangbeutel (2) und jeden Behälter (15) mit einem Identifikationsmittel (35) auszustatten, das Informationen enthält, die es nach der Trennung des Behälters (15) von dem Beutelsystem (1) ermöglichen, eindeutig festzustellen, dass der Probenahme-Behälter (15) und der Auffangbeutel (2) zu dem gleichen Beutelsystem (1) gehören.

20. Verfahren gemäß Anspruch 19, **dadurch gekennzeichnet, dass** es vorsieht, das Beutelsystem (1) sterilisiert in der Verpackung (36) anzuordnen und dann die Einheit eventuell zu pasteurisieren.

21. Verfahren gemäß Anspruch 19, **dadurch gekennzeichnet, dass** es vorsieht, das Beutelsystem (1) in der Verpackung (36) anzuordnen und dann die Einheit zu sterilisieren.

22. Verfahren gemäß Anspruch 19, **dadurch gekennzeichnet, dass** es vorsieht, das Beutelsystem (1) ohne die Behälter (15) zu sterilisieren, die Behälter (15) mit dem Beutelsystem zu verbinden, das genannte System zu verpacken und dann die Einheit eventuell zu pasteurisieren.
